(19) Europäisches Patentamt
European Patent Office
Office européen des brevets

(11) Veröffentlichungsnummer: **0 510 442 A1**

# (12) EUROPÄISCHE PATENTANMELDUNG

(21) Anmeldenummer: **92106138.8**

(51) Int. Cl.[5]: **C07D 213/89**, G03C 1/73

(22) Anmeldetag: **09.04.92**

(30) Priorität: **20.04.91 DE 4112967**

(43) Veröffentlichungstag der Anmeldung: **28.10.92 Patentblatt 92/44**

(84) Benannte Vertragsstaaten: **BE CH DE FR GB IT LI**

(71) Anmelder: **HOECHST AKTIENGESELLSCHAFT**
**Postfach 80 03 20**
**W-6230 Frankfurt am Main 80(DE)**

(72) Erfinder: **Lohaus, Gerhard, Dr.**
**Uhlandstrasse 4**
**W-6233 Klekheim(DE)**
Erfinder: **Spiess, Walter, Dr.**
**Rheingaustrasse 20**
**W-6110 Dieburg(DE)**
Erfinder: **Pawlowski, Georg. Dr.**
**Fritz-Kalle-Strasse 34**
**W-6200 Wiesbaden(DE)**

## (54) Substituted 1-sulfonyloxy-2-pyridones, process for their preparation and their use.

(57) Es werden 1-Sulfonyloxy-2-Pyridone der allgemeinen Formel I,

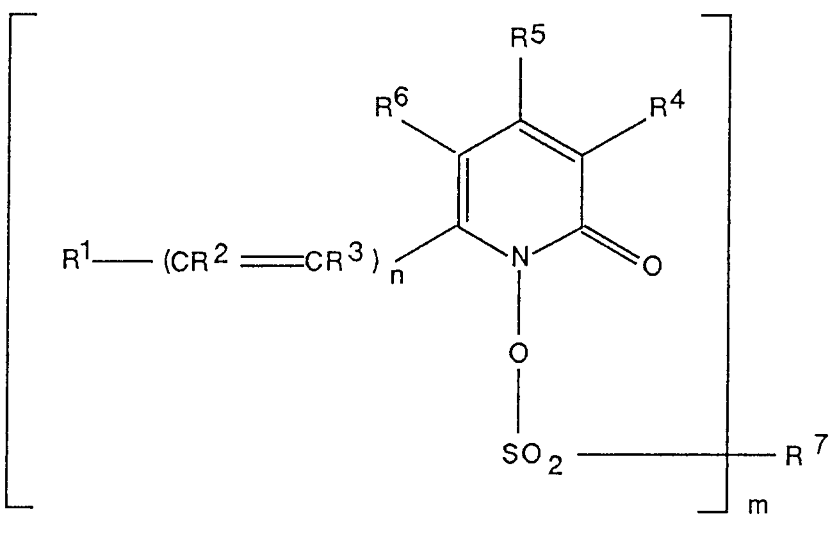

(I)

beschrieben, die eine gute Strahlungsempfindlichkeit über einen weiten Spektralbereich zeigen und daher als photoaktive Verbindungen in strahlungsempfindlichen Gemischen wertvoll sind .

EP 0 510 442 A1

Die Erfindung betrifft neue strahlungsempfindliche, in 6-Stellung chromophor substituierte 1-Sulfonyloxy2-pyridone, ein Verfahren zu ihrer Herstellung und ihre Verwendung, vorzugsweise in einem strahlungsempfindlichen Gemisch.

Die Verwendung von 1-Sulfonyloxy-2-pyridonen als Photooxidantien in strahlungsempfindlichen Gemischen, die einen Leukofarbstoff enthalten, ist aus der US-A 4 425 424 bekannt. In derartigen Gemischen bewirkt das Photooxidans bei der Bestrahlung beispielsweise eine Oxidation des Leukofarbstoffs, der dabei eine intensive Farbänderung eingeht und einen visuellen Kontrast zwischen belichteten und unbelichteten Bereichen hervorruft. Solche Farbumschläge sind in der Technik beispielsweise bei der Herstellung von Druckformen erwünscht, um nach der Belichtung bereits vor der Entwicklung das Kopierergebnis beurteilen zu können.

1-Benzolsulfonyloxy- und 1-(Toluol-4-sulfonyloxy)-2-pyridon sowie Verfahren zu deren Herstellung sind bekannt (E.C. Taylor et al., J. Org. Chem., 35, 1672, 1970). Die Strahlungsempfindlichkeit dieser Verbindungen wurde jedoch nicht erkannt.

Sulfonsäureester als Säurebildner in strahlungsempfindlichen Gemischen sind bekannt. Beispiele hierfür sind die Sulfonate von Hydroxymethylbenzoinderivaten (DE-A 19 19 678) oder 2-Nitrobenzylsulfonate (EP-A 0 330 386) und Pyrogallolsulfonate [T. Ueno et al., Chemical Amplification Positive Resist Systems Using Novel Sulfonates as Acid Generators, in "Polymers for Microelectronics - Science and Technology", Hrsg. Y. Tabata et al., Kodansha-Weinheim-New York, 1989, S. 66-67]. Derartige strahlungsempfindliche Sulfonsäureester sind aufgrund ihrer Absorptionseigenschaften besonders für die Bestrahlung mit energiereicher UV2-Strahlung (220 bis 280 nm) geeignet, während ihre Empfindlichkeit in der derzeit in der Technik verwendeten UV3- und UV4-Strahlung (350 bis 450 nm) unzureichend für praktische Anwendungen ist.

Verbindungen, die in diesem Bereich praxisgerechte Empfindlichkeiten aufweisen, sind aus der EP-A 0 137 452 bekannt. Dabei handelt es sich beispielsweise um Trichlormethylgruppen enthaltende Verbindungen, die bei der Belichtung Chlorradikale und Chlorwasserstoffsäuren bilden, die diesen Verbindungen die gewünschten Eigenschaften als Photoinitiatoren oder photosäuregeneratoren vermitteln. Die photolytisch gebildeten Halogenwasserstoffsäuren wirken jedoch stark korrodierend und können aus diesem Grund in vielen technischen Bereichen nicht eingesetzt werden.

Aufgabe der Erfindung war es daher, strahlungsempfindliche Verbindungen bereitzustellen, die insbesondere hohe Empfindlichkeiten im Bereich von Strahlung einer Wellenlänge zwischen 350 bis 450 nm oder im sichtbaren Bereich aufweisen und sich in einfacher Weise herstellen lassen. Gleichzeitig war es erwünscht, daß die erfindungsgemäßen Verbindungen auch gegenüber energiereicher UV-Strahlung, etwa der, die von KrF-Excimer-Lasern ausgesendet wird, empfindlich sind. Entsprechend der Aufgabe der Erfindung sollten diese Verbindungen bei der Bestrahlung reaktive, nicht korrodierend wirkende Photoprodukte bilden, so daß Photoinitiatoren und Photosäuregeneratoren bereitgestellt werden, die auch in Verbindung mit leicht korrodierbaren Materialien Anwendung finden können.

Gelöst wird die Aufgabe durch neue strahlungsempfindliche chromophor substituierte 1-Sulfonyloxy-2-pyridone der allgemeinen Formel I,

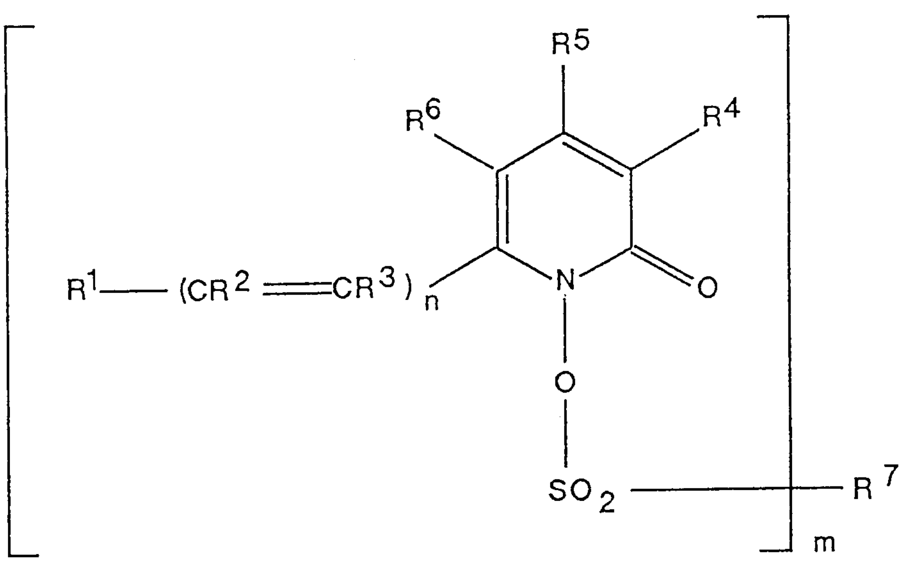

(I)

worin

| | |
|---|---|
| $R^1$ | einen Alkyl-, Cycloalkyl-, Aryl-, Aralkenyl-, Heteroaryl- oder Heteroaralkenylrest, |
| $R^2$ | Wasserstoff, Chlor, Brom, einen Alkyl-, Cycloalkyl-, Aryl- oder Heteroarylrest oder |
| $R^1$ und $R^2$ | gemeinsam einen fünf- bis achtgliedrigen Ring ausbilden, |
| $R^3$ | Wasserstoff oder einen Alkylrest, |
| $R^4$ | Wasserstoff, Halogen, Nitro, Acylamino, Cyan, Thiocyanato, einen Alkyl-, Alkenyl-, Alkinyl-, Aryl-, Aralkyl-, Alkylthio-, Arylthio- oder Cycloalkylthiorest, |
| $R^5$ | Wasserstoff, einen Alkyl- oder Arylrest oder |
| $R^4$ und $R^5$ | zusammen einen fünf- bis achtgliedrigen Ring ausbilden, |
| $R^6$ | Wasserstoff, Halogen, Nitro, Acylamino, Cyan, Thiocyanato, einen Alkyl-, Alkenyl-, Alkoxyalkyl-, Aryl-, Aralkyl-, Alkylthio-, Cycloalkylthio- oder Arylthiorest, |
| $R^7$ | einen Alkyl-, Cycloalkyl, per- oder hochfluorierten Alkylrest oder einen Aryl-, Arylalkyl- oder Heteroarylrest oder einen Alkylen- oder Arylenrest |
| m | die Zahl 1 oder 2 und |
| n | eine ganze Zahl von 1 bis 3 |

bedeuten.

Von den Verbindungen der allgemeinen Formel I sind solche bevorzugt, in denen

$R^1$ ein Alkyl- oder Cycloalkylrest oder ein Arylrest der allgemeinen Formel II

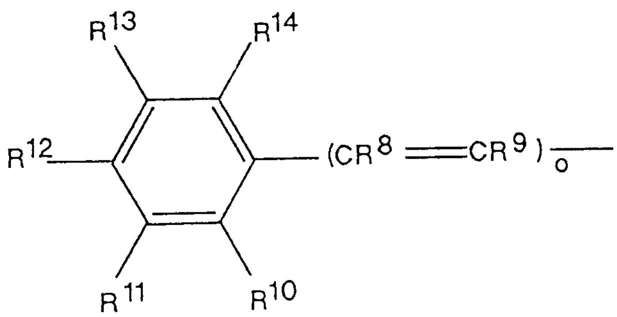

(II)

ist, worin

| | |
|---|---|
| $R^8$ und $R^9$ | gleich oder verschieden sind und Wasserstoff, eine niedere Alkyl- oder Arylgruppe darstellen, |
| $R^{10}$ bis $R^{14}$ | gleich oder verschieden sind und Wasserstoff, einen Alkyl-, Alkenyl-, Alkoxy-, Alkylthio-, Alkansulfonylrest mit jeweils bis zu 6 Kohlenstoffatomen, einen Cycloalkyloxy-, Cycloalkylthio-, Cycloalkansulfonylrest mit bis zu 8 Kohlenstoffatomen, gegebenenfalls am aromatischen Ring substituierten Phenyl-, Styryl-, Phenoxy-, Phenylthio-, Benzolsulfonyl-, Phenylalkoxy-, Phenylalkylthio-, Phenylalkansulfonylrest mit bis zu 3 Kohlenstoffatomen in der Alkylkette, Hydroxy, Halogen, Trifluoromethyl, Nitro, Cyan, Alkoxycarbonyl, Carbamoyl, das gegebenenfalls am Stickstoff durch einen oder zwei Alkylrest(e), die gegebenenfalls zu einem 5- bis 7-gliedrigen Ring verbunden sind, substituiert ist, Sulfamoyl, das gegebenenfalls am Stickstoff durch ein oder zwei Alkylrest(e), die gegebenenfalls zu einem 5- bis 7-gliedrigen Ring verbunden sind, substituiert ist, Alkansulfonyloxy, Arylsulfonyloxy, Acylamino, Alkylamino oder Arylamino bedeuten, |
| | oder zwei der Substituenten $R^{10}$ bis $R^{14}$, die einander benachbart sind, einen oder zwei weitere ankondensierte Ring(e) bilden, und |
| o | für die Zahl 0 oder 1 steht. |

Daneben sind auch solche Verbindungen der allgemeinen Formel I bevorzugt, in denen $R^1$ ein 5- oder 6-gliedriger Heterocyclus mit bis zu drei Heteroatomen der allgemeinen Formel III

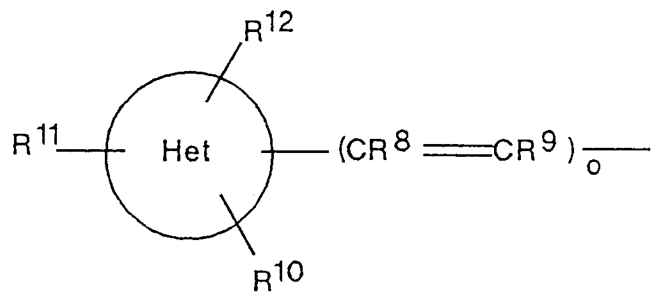

(III)

ist. Bevorzugte Verbindungen der allgemeinen Formel I sind schließlich auch solche, in denen

| | |
|---|---|
| $R^1$ | ein Ferrocenylrest und |
| $R^2$ | Wasserstoff, Chlor, Brom, Alkyl-, Cycloalkyl oder ein Rest der allgemeinen Formeln II oder III ist oder |
| $R^1$ und $R^2$ | gemeinsam einen fünf- bis achtgliedrigen Ring ausbilden, |
| $R^3$ | Wasserstoff oder ein Alkylrest, |
| $R^4$ | Wasserstoff, Halogen, Nitro, Acylamino, Cyan, Thiocyanato, ein Alkyl-, Aryl-, Alkylthio-, Arylthio- oder Cycloalkylthiorest, |
| $R^5$ | Wasserstoff, ein Alkyl- oder Arylrest ist oder |
| $R^4$ und $R^5$ | gemeinsam einen fünf- bis achtgliedrigen Ring ausbilden, |
| $R^6$ | Wasserstoff, Halogen, Nitro, Acylamino, Cyan, Thiocyanato, ein Alkyl-, Aryl-, Alkylthio-, Arylthio- oder Cycloalkylthiorest, |
| $R^7$ | ein Alkyl-, Cycloalkyl-, per- oder hochfluorierten Alkylrest oder ein Aryl-, Arylalkyl-, Aryl- oder Heteroarylrest oder ein Alkylen- oder Arylenrest ist und |
| m | für die Zahl 1 oder 2 und |
| n | für eine ganze Zahl von 1 bis 3 steht. |

Besonders bevorzugt sind solche Verbindungen der allgemeinen Formel I, worin $R^7$ ein Alkylrest mit 1 bis 4 Kohlenstoffatomen, ein hoch- oder perfluorierter Alkylrest mit 1 bis 4 Kohlenstoffatomen oder ein Arylrest der allgemeinen Formel IV

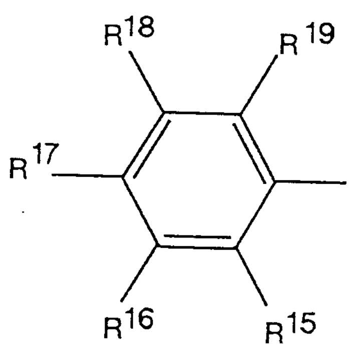

(IV)

ist, worin $R^{15}$ bis $R^{19}$ gleich oder verschieden sind und Wasserstoff- oder Halogenatome, die vorzugsweise Fluor, Chlor oder Brom sind, Alkylreste mit bis zu 6 Kohlenstoffatomen, die unsubstituiert oder durch Halogenatome, vorzugsweise Chlor oder Brom, Aryl- oder Aryloxyreste, substituiert sind und in denen einzelne Methylengruppen durch Sauerstoff- oder Schwefelatome ersetzt sein können und wobei jeweils zwei dieser Reste unter Ausbildung eines 5- oder 6-gliedrigen Rings verknüpft sein können, Cycloalkylreste mit bis zu 8 Kohlenstoffatomen, Alkenylreste mit bis zu 6 Kohlenstoffatomen, Aryl- oder Aryloxyreste mit bis zu 10 Kohlenstoffatomen bedeuten und die Gesamtzahl der Kohlenstoffatome der Reste $R^{15}$ bis $R^{19}$ maximal 12 beträgt.

Bevorzugt sind ebenfalls Verbindungen der allgemeinen Formel I, in denen $R^7$ ein Naphthyl- oder Heteroarylrest mit bis zu 10 Kohlenstoffatomen, ein Alkylenrest mit bis zu 6 Kohlenstoffatomen oder ein Arylen- oder Heteroarylenrest mit bis zu 14 Kohlenstoffatomen ist.

Ganz besonders bevorzugt sind solche Verbindungen der allgemeinen Formel I, worin

| | |
|---|---|
| $R^2$, $R^3$, $R^4$ und $R^6$ | ein Wasserstoffatom, |
| $R^5$ | eine Methylgruppe, |
| $R^7$ | einen Methyl-, Ethyl-, Trifluormethyl-, 1,1,2,3,3,3-Hexafluorpropyl-, Phenyl-, Tolyl-, 4-Fluorphenyl-, 4-Chlorphenyl-, 4-Bromphenyl oder 4-Nitrophenylrest und |
| m und n | jeweils die Zahl 1 |

bedeuten.

Im einzelnen kann unter den besonders bevorzugten Verbindungen der Rest $R^7$ z.B. die folgende Bedeutung haben:

Methyl, Ethyl, Propyl, Isopropyl, Butyl, Hexyl, Octyl, Decyl, Dodecyl, Hexadecyl, Octadecyl, 10-Camphyl, Chlormethyl, 2-Chlorethyl, 3-Chlorpropyl, Dichlormethyl, Trichlormethyl, Difluormethyl, Trifluormethyl, 2,2,2-Trifluorethyl, 3,3,3,2,1,1-Hexafluorpropyl, Perfluorhexyl, Trimethylsilylmethyl, Methansulfonylmethyl, Phenyl, Benzyl, 4-Acetylphenyl, 4-Acetylaminophenyl, 2-, 3- oder 4-Bromphenyl, 2-, 3- oder 4-Chlorphenyl, 2-, 3- oder 4-Fluorphenyl, 4-Jodphenyl, 2-Cyanphenyl, 4-Cyanphenyl, 2-, 3- oder 4-Methylphenyl, 4-Ethylyphenyl, 4-Propylphenyl, 4-Isopropylphenyl, 4-Isobutylphenyl, 4-tert.-Butylphenyl, 4-tert.-Amylphenyl, 4-Hexylphenyl, 4-Methoxyphenyl, 4-Butoxyphenyl, 4-Hexadecyloxyphenyl, 2-, 3- oder 4-Trifluormethylphenyl, 2- oder 4-Trifluormethoxyphenyl, 2-, 3- oder 4-Nitrophenyl, 3- oder 4-Carboxyphenyl, 2-Methoxycarbonylphenyl, 4-Tetrafluorethoxyphenyl, $\beta$-Styryl, 4-Acetylamino-3-chlorphenyl, 4-Acetylamino-3-fluorphenyl, 3,5-Bistrifluormethylphenyl, 2,5-Bis-(2,2,2-trifluorethoxy)phenyl, 2,5-Dimethylphenyl, 2,4-, 2,5- oder 3,4-Dimethoxyphenyl, 2,4-Diisopropylphenyl, 5-Brom-2-methoxyphenyl, 2- oder 3-Chlor-4-flüorphenyl, 3-Chlor-2-methylphenyl, 3-Chlor-4-methoxyphenyl, 2-Chlor-6-methylphenyl, 2-Chlor-4-trifluormethylphenyl, 5-Chlor-2-methoxyphenyl, 5-Fluor-2-methylphenyl, 2,5- oder 3,4-Dibromphenyl, 2,3-, 2,4- oder 2,5-, 2,6-, 3,4- oder 3,5-Dichlorphenyl, 2-(2,4-Dichlorphenoxy)phenyl, 4-(2-Chlor-6-nitrophenoxy)phenyl, 4-(3-Chlor-2-cyanphenoxy)phenyl, 2,4- oder 2,5-Difluorphenyl, 3-Carboxy-4-chlorphenyl, 4-Chlor-3-nitrophenyl, 2-Methyl-5-nitrophenyl, 4-Chlor-3- oder 2-Chlor-5-trifluormethylphenyl, 4-(2,2-Dichlorcyclopropyl)phenyl, 2,4-Dinitrophenyl, 4-Dimethylamino-3-nitrophenyl, 2-Nitro-4-trifluormethylphenyl, 2,4,6-Trimethylphenyl, 2,4,6-Triisopropylphenyl, 2,3,4-, 2,4,5- oder 2,4,6-Trichlorphenyl, 4-Chlor-2,5-dimethylphenyl, 2,4-Dichlor-5-methylphenyl, 3,5-Dichlor-2-hydroxyphenyl, 3,5-Dichlor-4-(4-nitrophenoxy)phenyl, 4-(2-Chlor-4-nitrophenoxy)-3,5-Dichlorphenyl, 4-Brom-2,5-difluorphenyl, 2,4-Dimethyl-3-nitrophenyl, 3,5-Dinitro-4-methylphenyl, 2,3,5,6-Tetramethylphenyl, 4-Methoxy-2,3,6-trimethylphenyl, 2,5-Dibrom-3,6-difluorphenyl, 2,3,4,5,6-Pentafluorphenyl, 1- oder 2-Naphthyl, 5-Diazo-6-oxo-5,6-dihydro-1-naphthyl, 6-Diazo-5-oxo-5,6-dihydro-1-naphthyl, 5-Diazo-6-oxo-5,6-dihydro-8-naphthyl, 5-Diazo-3-methoxy-6-oxo-5,6-dihydro-8-naphthyl, 5-Dimethylamino-1-naphthyl, 1-Anthracenyl, 2-Anthrachinonyl, 8-Chinolinyl, 2-Thienyl, 5-Chlor-2-thienyl, 4-Brom-2,5-dichlor-3-thienyl, 4,5-Dibrom-2-thienyl, 2,3-Dichlor-5-thienyl, 2-Brom-3-Chlor-5-thienyl, 3-Brom-2-chlor-5-thienyl, 3-Brom-5-chlor-2-thienyl, 2,5-Dichlor-3-thienyl, 2-(2-Pyridyl)-5-thienyl, 5-Chlor-1,3-dimethyl-4-pyrazolyl, 3,5-Dimethyl-4-isoxazolyl, 2,4-Dimethyl-5-thiazolyl, 2-Acetylamino-4-methyl-5-thiazolyl oder

1,4-Butylen, 2-Oxo-1,3-propylen, 1,2- oder 1,3-Phenylen, 3-Methyl-1,2-phenylen, 2,4,6-Trimethyl-1,3-phenylen, 4,4'-Biphenylen, 4,4'-Methylendiphenylen, 4,4'-Oxybiphenylen, 1,5-Naphthylen, 2-Chlor-3,5-thienylen, 2-(1-Methyl-5-trifluormethylpyrazol-3-yl)-3,5-thienylen.

Im einzelnen kann unter den besonders bevorzugten Verbindungen der Rest $R^1$ z.B. die folgende Bedeutung haben:

Methyl, Chlormethyl, Brommethyl, Ethyl, Propyl, Isopropyl, Butyl, Isobutyl, Hexyl, Cyclohexyl, Octyl, Phenyl, 4-Acetylaminophenyl, 4-Acetoxyphenyl, 3- oder 4-Benzyloxyphenyl, 3-Benzyloxy-4-methoxyphenyl, 4-Benzyloxy-3-methoxyphenyl, 4-Biphenyl, 3,5-Bis(trifluormethyl)phenyl, 2-, 3- oder 4-Fluorphenyl, 2-, 3- oder 4-Chlorphenyl, 2-,3- oder 4-Bromphenyl, 4-Jodphenyl, 2-, 3- oder 4-Cyanphenyl, 2-, 3- oder 4-Methylphenyl, 2-, 3- oder 4-Ethylphenyl, 2-, 3- oder 4-Propylphenyl, 2-, 3- oder 4-Isopropylphenyl, 2-, 3- oder 4-Butylphenyl, 2-, 3- oder 4-Isobutylphenyl, 2-, 3- oder 4-sec.-Butylphenyl, 2-, 3- oder 4-tert.-Butylphenyl, 2-, 3- oder 4-Pentylphenyl, 2-, 3- oder 4-Hexylphenyl, 2-, 3- oder 4-Cyclohexylphenyl, 2-, 3- oder 4-Nonylphenyl oder 2-, 3- oder 4-Dodecylphenyl, 2-, 3- oder 4-Methoxyphenyl, 2-, 3- oder 4-Ethoxyphenyl, 2-, 3- oder 4-Isopropoxyphenyl, 2-, 3- oder 4-Butoxyphenyl, 2-, 3- oder 4-Pentoxyphenyl, 2-, 3- oder 4-Octyloxyphenyl, oder 2-, 3- oder 4-Decyloxyphenyl, 2,3-, 2,4-, 2,6-, 3,4- oder 3,5- Difluorphenyl, 2,3-, 2,4-, 2,6-, 3,4- oder 3,5-Dichlorphenyl, 2,3-, 2,4-, 2,6-, 3,4- oder 3,5-Dibromphenyl, 3-(3,4-Dichlorphenoxy)phenyl, 3-(3,5-Dichlorphenoxy)phenyl, 3-Brom-4-fluorphenyl, 5-Brom-2,4-dimethoxyphenyl, 2-Chlor-6-fluorphenyl, 2-Chlor-5-, 2-Chlor-6-, 4-Chlor-3- oder 5-Chlor-2-nitrophenyl, 3-(4-Chlorphenoxy)phenyl, 3,4-Bisbenzyloxyphenyl, 2,3-, 2,4-, 2,5-, 3,4- oder 3,5-Dimethoxy-phenyl, 2,3-, 2,4-, 2,5-, 3,4- oder 3,5-Diethoxy-phenyl, 2,3-, 2,4-, 2,5-, 3,4- oder 3,5-Dibutoxy-phenyl oder 2,3-, 2,4-, 2,5-, 3,4- oder 3,5-Dihexyloxy-phenyl, 2,4-Dimethoxy-3-methyl-phenyl, 2-Ethoxy-5-methoxy-phenyl, 3-Chlor-4-methyl-phenyl, 2,4- oder 2,5-Dimethylphenyl, 2-, 3- oder 4-Methoxyethyl-phenyl, 2-, 3- oder 4-Ethoxyethyl-phenyl, 2-, 3- oder 4-Butoxyethylphenyl, 2,6-Dinitro-phenyl, 2,4,6-Trimethylphenyl, 3,4,5-Trimethoxy-phenyl, 3,4,5-Triethoxy-phenyl, 2,3- oder

3,4-Methylendioxyphenyl, 2- oder 3-Thienyl, 2-Fluorenyl, 9-Anthryl, 1-Pyrenyl, 9-Phenanthryl, 5-Brom-2-thienyl, 3-Methyl-2-thienyl, 5-Methyl-2-thienyl, 5-Nitrofuryl, 10-Chlor-9-anthryl oder Ferrocenyl.

Diese Reste sind besonders deshalb bevorzugt, weil die ihnen zugrundeliegenden Ausgangsverbindungen kommerziell erhältlich sind. Weitere Verbindungen sind aber auf einfache Weise nach dem Fachmann geläufigen Methoden herstellbar.

Die erfindungsgemäßen Verbindungen bilden unter Einwirkung von aktinischer Strahlung reaktive Zwischenstufen, die zur Einleitung spezieller chemischer Reaktionen, beispielsweise radikalischer Polymerisationen, befähigt sind. Sie bilden bei ihrer Bestrahlung jedoch insbesondere organische Säuren, die Reaktionen, wie kationische Polymerisationen, Vernetzungen oder Spaltungen säurelabiler Verbindungen katalysieren oder mit Basen reagieren können, was sich z.B. in einem Farbumschlag bei Indikatorfarbstoffen äußern kann.

Die oben aufgeführten Verbindungen haben Absorptionsmaxima im Bereich zwischen 200 bis 550 nm und sind daher für eine Bestrahlung mit energiereicher UV-Strahlung im Bereich der UV2- (220 bis 280 nm), UV3- (300 bis 350 nm) oder UV4- (350 bis 450 nm) Strahlung sowie mit energiereichem sichtbaren Licht (450 bis 550 nm) sehr gut geeignet. Sie zeigen auch bei Bestrahlung mit Strahlung einer Wellenlänge $< 220$ nm hohe Aktivitäten; sind aber wegen ihrer hohen Absorption in diesem Bereich bevorzugt für sogenannte "Toplayer Imaging" Verfahren anwendbar.

Gegenstand der Erfindung ist weiterhin ein Verfahren zur Herstellung der neuen chromophor substituierten 1-Sulfonyloxy-2-pyridone der allgemeinen Formel I, das anhand des Schemas 1 dargestellt ist.

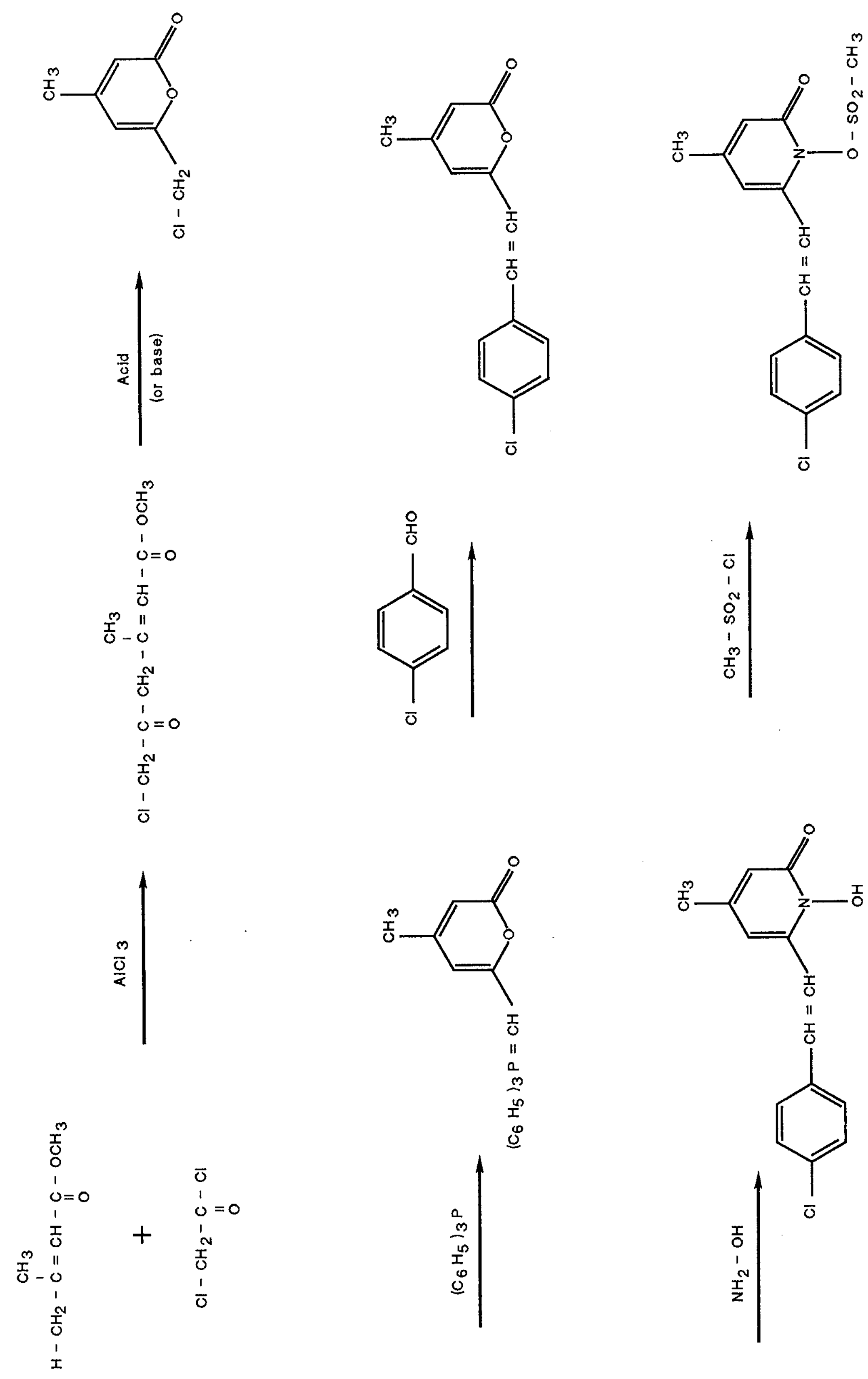


Gemäß diesem Schema wird

a) ein 6-Halogenomethyl-2-pyron in eine Phosphonium- oder Phosphonoverbindung überführt,

b) diese einer Wittig-Reaktion oder deren Varianten unterworfen,

c) die erhaltene Verbindung in ein 1-Hydroxy-2-pyridon umgewandelt und

d) mit einem Sulfonsäurehalogenid zu einem 1-Sulfonyloxy-2-pyridon der allgemeinen Formel I umgesetzt.

Als Sulfonsäurehalogenid wird ein Sulfonsäurechlorid eingesetzt. Die Umsetzung mit dem Sulfonsäurehalogenid wird zweckmäßig in Gegenwart einer organischen Base in einem organischen Lösemittel bei Temperaturen zwischen 0 und 20°C durchgeführt.

Die neuen chromophor substituierten 1-Sulfonyloxy-2-pyridone der allgemeinen Formel I lassen sich in herausragender Weise als Photoinitiatoren oder Photosäuregeneratoren für strahlungsempfindliche Aufzeichnungsmaterialien verwenden. Ihr Einsatz in positiv und negativ arbeitenden strahlungsempfindlichen Gemischen wird in den nachstehend beschriebenen Anwendungsbeispielen 1 bis 4 erläutert; eine detaillierte Beschreibung ihrer Verwendungsmöglichkeiten enthalten die gleichzeitig eingereichten deutschen Patentanmeldungen P 41 12 966.0 und P 41 12 965.2.

Die nachstehend beschriebenen Beispiele illustrieren die Erfindung, sollen aber nicht beschränkend wirken. Im Folgenden steht Gt für Gewichtsteile und Vt für Volumenteile, die sich zu Gewichtsteilen wie g zu $cm^3$ verhalten.

## Herstellungsbeispiele

Die Beispiele 1 bis 5 (Verbindungen 1 bis 5) verdeutlichen die bevorzugten Methoden zur Herstellung der erfindungsgemäßen chromophor substituierten 1-Sulfonyloxy-2-pyridone der allgemeinen Formel I. Dem Fachmann ist ohne weiteres ersichtlich, daß sich diese Herstellungsmethoden auch auf die Verbindungen 6 bis 109 anwenden lassen und sich auch durch diese Auswahl noch keine Begrenzung des Erfindungsgedankens abzeichnet. Anhand der Anwendungsbeispiele 1 bis 4 werden einige typische Anwendungsmöglichkeiten der erfindungsgemäßen Verbindungen der allgemeinen Formel I erläutert. Im ersten Herstellungsbeispiel wird dabei der gesamte Syntheseweg, von großtechnisch zugänglichen Ausgangsprodukten ausgehend, beschrieben. Über die ersten Stufen dieser Synthese wurde von G. Lohaus et al., Chem. Ber., 100, 658, 1967 bereits eingehend berichtet. Eine große Zahl verschiedenartig substituierter 1-Hydroxy-2-pyridone und Methoden zu ihrer Herstellung sind in Arzneim. Forsch./Drug Res., 31 (II), 8a, 1311 (1981) beschrieben worden.

### Herstellungsbeispiel 1

1. Stufe: Zu einer Suspension von 532 Gt Aluminiumtrichlorid in 250 Vt Ethylenchlorid und 282 Gt Chloracetylchlorid wurden bei 50 bis 60°C 230 Gt 3-Methylcrotonsäure-methylester so zugegeben, daß die Temperatur gerade gehalten wurde. Anschließend wurde eine Stunde auf 65 bis 70°C erhitzt und auf Eis gegossen. Die wäßrige Phase wurde mit Methylenchlorid extrahiert und die organische Phase mit Wasser und gesättigter Natriumhydrogencarbonatlösung gewaschen. Nach Trocknung über Natriumsulfat wurde fraktioniert destilliert. Ausbeute: 280 Gt (74%), Sdp. 120 bis 122°C/5 Torr.

2. Stufe: 382 Gt des vorstehend beschriebenen Produkts wurden in einer Mischung aus 1000 Vt Eisessig und 10 Vt konzentrierter Schwefelsäure gelöst und zum Sieden erhitzt. Über eine kurze Kolonne wurde die berechnete Menge Methylacetat abdestilliert. Anschließend wurde der Eisessig im Vakuum entfernt und der Rückstand in Eiswasser gegeben, wobei das Pyron kristallisierte. Zur weiteren Reinigung kann das abgetrennte Produkt bei 116 bis 117°C/2 Torr destilliert werden. Nach Umkristallisation aus Tetrachlorkohlenstoff erhielt man 296 Gt 6-Chlormethyl-4-methyl-2-pyron. Ausbeute: 93%, Fp. 78°C.

3. Stufe: 320 Gt des vorstehend beschriebenen Produkts wurden zusammen mit 540 Gt Triphenylphosphin und 1500 Vt Acetonitril 8 Stunden zum Rückfluß erhitzt. Die Mischung wurde in Eis gekühlt und das ausgefallene Produkt abgesaugt. Die Mutterlauge wurde eingeengt, wobei weiteres Produkt ausfiel, das abgesaugt wurde. Es wurden 799 Gt (4-Methyl-6-oxo-6H-pyron-2-ylmethyl)-triphenyl-phosphoniumchlorid erhalten.

4. Stufe: 106 Gt des vorstehend beschriebenen Produkts wurden zusammen mit 35,5 Gt 4-Chlorbenzaldehyd in 200 Vt Methanol gelöst. Dazu wurde eine Lösung aus 6 Gt Natrium in 150 Vt Methanol gegossen, wobei sich die Mischung vorübergehend rot färbte und stark exotherm reagierte. Es wurde 6 Stunden bei Raumtemperatur gerührt, auf -25°C gekühlt und das ausgefallene Produkt abgesaugt. Dieses wurde mit gekühltem Methanol gewaschen, mit Wasser verrührt und erneut abgesaugt. Es wurden 26 Gt 6-(4-Chlor-styryl)-4-methyl-2-pyron erhalten, das aus Acetontril umkristallisiert werden kann. Fp. 144°C.

5. Stufe: 22,5 Gt des vorstehend beschriebenen Produkts wurden mit 9 Gt Hydroxylaminhydrochlorid und 110 Gt 2-Aminopyridin auf etwa 70°C erwärmt. Die Mischung wurde 72 Stunden bei dieser Temperatur belassen, wobei nach 3, 18, 30 und 42 Stunden jeweils 3,3 Gt Hydroxylaminhydrochlorid nachdosiert wurden. Die erkaltete Mischung wurde in Methylenchlorid aufgenommen und mit verdünnter

Salzsäure und Wasser gewaschen. Die organische Phase wurde in einer Lösung aus 10 Gt Natriumhydroxid in 700 Vt entionisiertem Wasser aufgerührt, das entstandene Natriumsalz abfiltriert und in 600 Vt heißem Wasser gelöst. Durch Zugabe von Eisessig wurde die wäßrige Lösung auf pH 5 eingestellt und abgekühlt. Das dabei kristallisierende Rohprodukt wurde abgenutscht und getrocknet. Es wurden 15 Gt eines Rohprodukts mit einem Schmelzpunkt von 195°C erhalten, das aus Methylenchlorid umkristallisiert wurde. Es wurden gelbliche Kristalle [6-(4-Chlor-styryl)-1-hydroxy-4-methyl-2-pyridon] mit einem schmelzpunkt von 205°C erhalten.

| | | | | |
|---|---|---|---|---|
| Analyse: (261,70) | ber.<br>gef. | C 64,25%<br>C 64,2 % | H 4,62%<br>H 4,6 % | N 5,35%<br>N 5,4 % |

6. Stufe: Zu einer Lösung aus 4 Gt des vorstehend beschriebenen Produkts und 20 Gt Methansulfonylchlorid in 50 Vt Methylenchlorid wurden 1,85 Gt Triethylamin bei 10°C in 20 Vt Methylenchlorid zugetropft. Die Lösung wurde 24 Stunden bei Raumtemperatur nachgerührt, mit Wasser ausgeschüttelt und das Lösemittel abrotiert. Das kristalline Produkt wurde getrocknet, auf eine Silicagelsäule gegeben und mit Methylenchlorid eluiert. Die vereinigten Produktfraktionen ergaben nach Einengen 4,2 Gt 6-(4-Chlor-styryl)-1-methansulfonyloxy-4-methyl-2-pyridon mit einem Zersetzungspunkt von 190°C. Eine Probe wurde aus Acetonitril umkristallisiert und ergab ein Produkt mit einem Zersetzungspunkt von 193°C .

| | | | | |
|---|---|---|---|---|
| Analyse: (339,77) | ber.<br>gef. | C 53,02%<br>C 52,7% | H 4,15%<br>H 4,4% | N 4,12%<br>N 4,2% |

Herstellungsbeispiel 2

1. Stufe: Zu 79,3 Gt 6-Chlormethyl-4-methyl-2-pyron (s. Herstellungsbeispiel 1, 2. Stufe) wurden unter Rühren in einer Destillationsapparatur bei 100°C langsam 108 Gt Triethylphosphit zugetropft. Dabei destillierte Ethylchlorid in die Vorlage über. Nachdem die Ethylchlorid-Abspaltung schwächer wurde, wurde die Badtemperatur langsam auf 130°C erhöht und etwa 6 Stunden bei dieser Temperatur nachgerührt. Nach Ablauf wurden weitere 320 Gt 6-Chlormethyl-4-methyl-2-pyron zur Mischung hinzugefügt und langsam 432 Gt Triethylphosphit bei einer Badtemperatur von 140°C zugetropft. Es wurde erneut 9 Stunden bei dieser Temperatur nachgerührt. Nach dem Abkühlen wurden dann anschließend flüchtige Bestandteile der Mischung bei 70°C im Ölpumpenvakuum etwa 3 Stunden abdestilliert. Es verblieben 610 Gt 6-Diethoxyphosphorylmethyl-4-methyl-2-pyron als zäher Rückstand, der nach einiger Zeit zu kristallisieren begann.

2. Stufe: Zu einer Mischung aus 45,5 Gt Benzophenon, 65 Gt der vorstehend beschriebenen Verbindung und 200 Vt 1,2-Dimethoxyethan wurde unter Inertgas und intensivem Rühren 7,75 Gt Natriumhydrid (80% Suspension) zugegeben und die Mischung 24 Stunden bei 70°C gerührt. Das Gemisch wurde in Methylenchlorid aufgenommen, mehrmals mit Wasser gewaschen, getrocknet und am Rotationsverdampfer eingeengt. Man erhielt 74,3 Gt eines aus zwei Komponenten bestehenden Rohprodukts, das über eine Kieselgelsäule mit Methylenchlorid als Laufmittel aufgetrennt wurde. Die Fraktionen, die das Produkt mit der höheren Retentionszeit enthielten, wurden vereint und eingeengt. Man erhielt 55 Gt kristallines 6-(2,2-Diphenyl-vinyl)-4-methyl-2-pyron, das aus Hexan umkristallisiert werden kann.

3. Stufe: 20 Gt des vorstehend beschriebenen Produkts wurden mit 5,6 Gt Hydroxylaminhydrochlorid und 80 Gt Aminopyridin auf etwa 75°C erwärmt. Die Mischung wurde 58 Stunden bei dieser Temperatur belassen, wobei nach 6, 22 und 32 Stunden jeweils 2,8 Gt Hydroxylaminhydrochlorid nachdosiert wurden. Die erkaltete Mischung wurde in Methylenchlorid aufgenommen und mit verdünnter Salzsäure und Wasser gewaschen, getrocknet und eingeengt. Der mit 22 Gt anfallende Rückstand wurde aus Acetonitril umkristallisiert. Es wurden 14 Gt eines gelben Produkts [6-(2,2-Diphenyl-vinyl)-1-hydroxy-4-methyl-2-pyridon] mit einem Schmelzpunkt von 169°C erhalten.

| | | | | |
|---|---|---|---|---|
| Analyse: (303,26) | ber.<br>gef. | C 79,18%<br>C 79,0% | H 5,65%<br>H 5,8% | N 4,62%<br>N 4,5% |

4. Stufe: Zu einer Lösung aus 8 Gt des vorstehend beschriebenen Produkts und 3,5 Gt Methansulfonylchlorid in 70 Vt Methylenchlorid wurden bei 5 bis 10°C 3 Gt Triethylamin in 20 Vt Methylenchlorid zugetropft. Die Mischung wurde 24 Stunden bei Raumtemperatur nachgerührt, mit Wasser ausgeschüttelt und das Lösemittel abrotiert. Der etwa 12,5 Gt betragende viskose Rückstand kristallisierte beim Stehenlassen und wies einen Schmelzpunkt von etwa 130°C auf. Das Rohprodukt wurde über eine Silicagelsäule mit Methylenchlorid eluiert. Die vereinigten Produktfraktionen ergaben nach Einengen 8,2 Gt 6-(2,2-Diphenyl-vinyl)-1-methansulfonyloxy-4-methyl-2-pyridon mit einem Schmelzpunkt von 140°C.

| | | | | |
|---|---|---|---|---|
| Analyse: (381,44) | ber.<br>gef. | C 66,12%<br>C 65,7% | H 5,02%<br>H 5,1% | N 3,67%<br>N 3,4% |

Herstellungsbeispiel 3

Stufe 1: 85 Gt (4-Methyl-6-oxo-6H-pyron-2-ylmethyl)triphenyl-phosphoniumchlorid (s. Herstellungsbeispiel 1, 3. Stufe) und 41,2 Gt Anthracen-9-carbaldehyd wurden in 200 Vt Methanol dispergiert. Dazu wurde unter Rühren ziemlich rasch eine Lösung von 5 Gt Natrium in 100 Vt Methanol zugegeben, wobei sich die Mischung erwärmte. Es wurde 1 Stunde bei etwa 50°C, anschließend 72 Stunden bei Raumtemperatur weitergerührt, anschließend auf -25°C abgekühlt und das ausgefallene Produkt abgesaugt. Das Produkt wurde mit Methanol gewaschen, dann mit Wasser verrührt und erneut abgesaugt. Es wurden 52 Gt 6-(2-Anthracen-9-yl-vinyl)-4-methyl-2-pyron mit einem Schmelzpunkt von etwa 200°C erhalten, das aus Acetonitril umkristallisiert einen Schmelzpunkt von 203°C aufwies.
Stufe 2: 20 Gt des vorstehend beschriebenen Pyrons wurden mit 5,6 Gt Hydroxylaminhydrochlorid und 80 Gt 2-Aminopyridin auf 70°C erwärmt. Die Mischung wurde 72 Stunden bei dieser Temperatur belassen, wobei nach 7, 23 und 32 Stunden jeweils 2,8 Gt Hydroxylaminhydrochlorid nachdosiert wurden. Die erkaltete Mischung wurde in Methylenchlorid aufgenommen und mit verdünnter Salzsäure und Wasser gewaschen. Dabei fiel ein Teil des Produkts aus. Die organische Phase wurde am Rotationsverdampfer eingeengt. Der Rückstand wurde aus Dimethylformamid (DMF) umkristallisiert. Die erhaltenen Kristalle wurden zusammen mit dem ausgefallenen Produkt erneut aus DMF umkristallisiert und mit Methanol gewaschen. Man erhielt 12,6 Gt eines analysenreinen orangefarbenen Pulvers [6-(2-Anthracen-9-yl-vinyl)-1-hydroxy-4-methyl-2-pyridon], das einen Schmelzpunkt > 260°C aufwies.

| | | | | |
|---|---|---|---|---|
| Analyse: (327,39) | ber.<br>gef. | C 80,71%<br>C 80,9 % | H 5,24%<br>H 5,4 % | N 4,28%<br>N 4,3 % |

3. Stufe: 4,5 Gt der vorstehend beschriebenen Verbindung und 1,83 Gt Methansulfonylchlorid wurden in 50 Vt Methylenchlorid gelöst und auf 5°C gekühlt. Dazu wurde eine Mischung von 1,6 Gt Triethylamin in 20 Vt Methylenchlorid langsam zugetropft. Es wurde 3 Tage bei Raumtemperatur nachgerührt. Die organische Phase wurde mit Wasser gewaschen, getrocknet und das Lösemittel am Rotationsverdampfer abgezogen. Der Rückstand wurde erneut in Methylenchlorid aufgenommen und über eine Kieselgelsäule mit Methylenchlorid als Laufmittel eluiert. Die deutlich erkennbare Hauptfraktion wurde gesammelt, vom Lösemittel befreit und das verbliebene schaumartige Produkt mit warmen Diisopropylether verrührt. Nach Trocknung wurden 1,9 Gt 6-(2-Anthracen-9-yl-vinyl)-1-methansulfonyloxy-4-methyl-2-pyridon erhalten, das sich bei einer Temperatur von 180°C unter Verpuffung zersetzte.

| | | | | |
|---|---|---|---|---|
| Analyse: (405,48) | ber.<br>gef. | C 68,13%<br>C 68,1 % | H 4,72%<br>H 4,9 % | N 3,46%<br>N 3,3 % |

Herstellungsbeispiel 4

Stufe 1: 26 Gt 6-Diethoxyphosphorylmethyl-4-methyl-2-pyron (s. Herstellungsbeispiel 2, 1. Stufe) und 17,4 Gt 4-Trifluormethylbenzaldehyd wurden in 80 Vt 1,2-Dimethoxyethan gelöst. Unter Stickstoff wurden dann portionsweise 3,2 Gt einer 80% Natriumhydrid-Dispersion derart zugefügt, daß sich die Reaktionsmischung nicht über 50°C erwärmte. Sie wurde 18 Stunden bei dieser Temperatur nachgerührt und nach dem Abkühlen mit Methylenchlorid verdünnt. Die Lösung wurde mehrmals mit Wasser gewaschen,

getrocknet und eingeengt. Es verblieben 26,9 Gt eines kristallinen Pulvers, das über eine Kieselgelsäule mit Methylenchlorid als Laufmittel eluiert wurde. Aus der Hauptfraktion wurden nach Einengen 18,9 Gt eines Pulvers vom Schmelzpunkt 155°C erhalten, das sich als das gewünschte analysenreine 4-Methyl-6-(4-trifluormethyl-styryl)-2-pyron erwies.

Stufe 2: 15 Gt des vorstehend beschriebenen Produkts, 45 Gt Imidazol, 5 Gt N-Methylpyrrolidon und 5,6 Gt Hydroxylamin-hydrochlorid wurden unter Rühren 55 Stunden auf 75°C erwärmt. Nach 8, 24 und 32 Stunden Reaktionszeit wurden je 2,8 Gt Hydroxylamin-hydrochlorid nachdosiert. Die erkaltete Mischung wurde in Methylenchlorid aufgenommen, mit Wasser gewaschen, getrocknet und eingeengt. Es wurden 14,6 Gt eines Rohprodukts erhalten, das aus Ethylenglykolmonomethylether umkristallisiert wurde. Das auskristallisierte 1-Hydroxy-4-methyl-6-(4-trifluoromethyl-styryl)-2-pyridon wies einen Schmelzpunkt von 230°C auf.

| | | | | | |
|---|---|---|---|---|---|
| Analyse: (295,28) | ber.<br>gef. | C 61,02%<br>C 61,3 % | H 4,10%<br>H 4,1 % | F 19,30% | N 4,74% |

Stufe 3: 1,8 Gt der vorstehend beschriebenen Verbindung und 1,9 Gt 4-Brombenzolsulfonylchlorid wurden in 40 Vt Methylenchlorid gelöst und auf 5°C gekühlt. Dazu wurde eine Mischung von 1,0 Gt Triethylamin in 5 Vt Methylenchlorid langsam zugetropft. Es wurde 66 Stunden bei Raumtemperatur nachgerührt. Die organische Phase wurde mit Wasser gewaschen, getrocknet und das Lösemittel am Rotationsverdampfer abgezogen. Der Rückstand (3,2 Gt) wurde erneut in Methylenchlorid aufgenommen und über eine Kieselgelsäule mit Methylenchlorid/Methanol (99/1) als Laufmittel eluiert. Die deutlich erkennbare Hauptfraktion wurde gesammelt, vom Lösemittel befreit und das verbliebene schaumartige Produkt mit warmen Diisopropylether verrührt, wobei Kristallisation des Produkts eintrat. Nach Trocknung wurden 2,2 Gt 1-(4-Brombenzolsulfonyloxy)-4-methyl-6-(4-trifluormethyl-styryl)-2-pyridon, das bei einer Temperatur von 158°C schmolz, erhalten.

| | | | | | |
|---|---|---|---|---|---|
| Analyse: (514,33) | ber.<br>gef. | C 49,04%<br>C 48,6 % | H 2,94%<br>H 3,1 % | N 2,72%<br>N 2,7 % | F 11,08% |

<u>Herstellungsbeispiel 5</u>

Stufe 1: 32,5 Gt 6-Diethoxyphosphorylmethyl-4-methyl-2-pyron (s. Herstellungsbeispiel 2, 1. Stufe) und 30 Gt 4-(4-Chlorphenylmercapto)benzaldehyd wurden in 120 Vt Methanol dispergiert. Unter Stickstoff wurde eine Lösung aus 3,1 Gt Natrium in 120 Vt Methanol zugetropft, wobei die Mischung exotherm reagierte und nach kurzzeitiger Ausbildung einer klaren Lösung eine voluminöse Ausfällung entstand. Das Gemisch wurde 22 Stunden bei Raumtemperatur nachgerührt, mit Eis gekühlt und abgesaugt. Der praktisch analysenreine Rückstand wurde mit vorgekühltem Methanol gewaschen und getrocknet. Es wurden 35 Gt des 6-[4-(4-Chlor-phenylmercapto)styryl]-4-methyl-2-pyron erhalten, das bei 164°C schmolz.

Stufe 2: 20 Gt des vorstehend beschriebenen Produkts, 80 Gt 2-Aminopyridin und 5,6 Gt Hydroxylamin-hydrochlorid wurden unter Rühren 83 Stunden auf 75°C erwärmt. Nach 18, 27 und 34 Stunden Reaktionszeit wurden je 2,8 Gt Hydroxylamin-hydrochlorid nachdosiert. Die erkaltete Mischung wurde in Methylenchlorid aufgenommen und mit verdünnter Salzsäure und Wasser gewaschen, getrocknet und eingeengt. Es wurden 21,8 Gt eines Rohprodukts erhalten, das aus Ethylenglykolmonomethylether umkristallisiert wurde. Das auskristallisierte 6-[4-(4-Chlor-phenylmercapto)styryl]-1-hydroxy-4-methyl-2-pyron wies einen Schmelzpunkt von 210°C auf.

| | | | | | |
|---|---|---|---|---|---|
| Analyse: (369,88) | ber.<br>gef. | C 64,95%<br>C 65,1% | H 4,36%<br>H 4,5% | Cl 9,58% | N 3,79% |

Stufe 3: 3,45 Gt der vorstehend beschriebenen Verbindung und 2,15 Gt 4-Fluorbenzolsulfonylchlorid wurden in 40 Vt Methylenchlorid gelöst und auf 5°C gekühlt. Dazu wurde eine Mischung von 1,3 Gt Triethylamin in 10 Vt Methylenchlorid langsam zugetropft. Es wurde 66 Stunden bei Raumtemperatur nachgerührt. Die organische Phase wurde mit Wasser gewaschen, getrocknet und das Lösemittel am

Rotationsverdampfer abgezogen. Der viskose Rückstand (5,1 Gt) wurde erneut in Methylenchlorid aufgenommen und über eine Kieselgelsäule mit Methylenchlorid/Methanol (99/1) als Laufmittel eluiert. Die deutlich erkennbare Hauptfraktion wurde gesammelt, vom Lösemittel befreit und das verbliebene schaumartige Produkt mit warmen Diisopropylether verrührt, wobei Mikrokristallisation des Produkts eintrat. Das Produkt wurde aus Acetontril umkristallisiert. Nach Trocknung wurden 2,0 Gt an 6-[4-(4-Chlor-phenylmercapto)-styryl]-1-(4-fluor-benzolsulfonyloxy)-4-methyl-2-pyridon erhalten, das bei einer Temperatur von 150°C schmolz.

| | | | | | |
|---|---|---|---|---|---|
| Analyse: (528,03) | ber.<br>gef. | C 59,14%<br>C 59,2 % | H 3,63%<br>H 3,7 % | N 2,65%<br>N 2,6 % | F 3,60% |

Verbindungen 6 bis 109

Nachstehend sind weitere erfindungsgemäße Verbindungen aufgeführt, die entsprechend den vorstehend beschriebenen Herstellungsbeispielen darstellbar sind:

6) 6-(4-Fluor-styryl)-1-methansulfonyloxy-4-methyl-2-pyridon
7) 1-Benzolsulfonyloxy-4-methyl-6-styryl-2-pyridon
8) 6-(3,4-Dimethoxy-styryl)-1-ethansulfonyloxy-4-methyl-2-pyridon
9) 6-(4-Methoxy-styryl)-4-methyl-1-(4-nitro-benzolsulfonyloxy)-2-pyridon
10) 6-(4-Cyan-styryl)-4-methyl-1-trifluormethansulfonyloxy)-2-pyridon
11) 6-(3-Benzyloxy-styryl)-1-(4-chlor-benzolsulfonyloxy)-4-methyl-2-pyridon
12) 1-Butansulfonyloxy-6-(3,4-dichlor-styryl)-4-methyl-2-pyridon
13) 1-(4-Chlor-3-nitro-benzolsulfonyloxy)-6-(3-ethoxy-6-methoxy-styryl)-4-methyl-2-pyridon
14) 1-(4-Brom-benzolsulfonyloxy)-6-(3-chlor-4-methylstyryl)-4-methyl-2-pyridon
15) 6-(4-Benzyloxy-3-methoxy-styryl)-1-(4-chlor-benzolsulfonyloxy)-4-methyl-2-pyridon
16) 1-Isopropansulfonyloxy-6-(4-phenyl-buta-1,3-dienyl)-4-methyl-2-pyridon
17) 4-Methyl-6-(2-naphthalen-1-yl-vinyl)-1-(3-nitrobenzolsulfonyloxy)-2-pyridon
18) 6-[4-(2-Ethoxy-ethoxy)-styryl]-1-methansulfonyloxy-4-methyl-2-pyridon
19) 6-(2,4-Dimethoxy-3-methyl-styryl)-1-(toluol-4-sulfonyloxy)-4-methyl-2-pyridon
20) 4-Methyl-6-styryl-1-trichlormethansulfonyloxy-2-pyridon
21) 4-Methyl-6-(2-naphthalin-2-yl-vinyl)-1-(toluol-4-sulfonyloxy)-2-pyridon
22) 4-Cyclohexyl-1-methansulfonyloxy-6-(4-methoxy-styryl)-2-pyridon
23) 3-Cyan-6-(3,4-dimethoxy-styryl)-4-methyl-1-(toluol-4-sulfonyloxy)-2-pyridon
24) 6-[2-(4-Chlor-phenyl)-prop-1-enyl]-1-methansulfonyloxy-4-methyl-2-pyridon
25) 6-(3-Chlor-4-methoxy-styryl)-1-(4-nitro-benzolsulfonyloxy)-4-phenyl-2-pyridon
26) 4-Butyl-6-(3,4-dimethoxy-styryl)-1-(4-methoxy-benzolsulfonyloxy)-2-pyridon
27) 6-[2,2-Bis-(4-methoxy-phenyl)-vinyl]-4-methyl-1-(toluol-4-sulfonyloxy)-2-pyridon
28) 6-(2-Anthracen-9-yl-vinyl)-4-methyl-1-(toluol-4-sulfonyloxy)-2-pyridon
29) 1-Benzolsulfonyloxy-5-chlor-4-ethyl-6-(4-methoxystyryl)-2-pyridon
30) 4-Methyl-3-nitro-6-(2-thiophen-2-yl-vinyl)-1-(toluol-4-sulfonyloxy)-2-pyridon
31) 3-Butylmercapto-1-methansulfonyloxy-6-(4-methoxystyryl)-4-methyl-2-pyridon
32) 3,5-Dibrom-4-methyl-6-(3-methyl-styryl)-1-(toluol-4-sulfonyloxy)-2-pyridon
33) 1-Ethansulfonyloxy-3-methoxymethyl-4-methyl-6-(2-naphthalin-2-yl-vinyl)-2-pyridon
34) 3,5-Dichlor-6-[2-(6-methoxy-naphthalin-2-yl)-vinyl]-4-methyl-1-(toluol-4-sulfonyloxy)-2-pyridon
35) 1-Methansulfonyloxy-4-methyl-6-(4-phenyl-styryl)-2-pyridon
36) 1-Benzolsulfonyloxy-3-brom-6-[4-(4-chlor-phenoxy)-styryl]-4-methyl-2-pyridon
37) 6-[4-(4-Chlor-phenylmercapto)-styryl]-1-(4-fluorbenzolsulfonyloxy)-4-methyl-2-pyridon
38) 6-[4-(4-Chlor-benzyloxy)-styryl]-1-(4-isopropylbenzolsulfonyloxy)-4-methyl-2-pyridon
39) 6-(4-Chlor-3-trifluormethyl-styryl)-1-(1,1,2,3,3,3-hexafluorpropansulfonyloxy)-4-methyl-2-pyridon
40) 1-Benzolsulfonyloxy-6-(4-dimethylamino-styryl)-4-methyl-2-pyridon
41) 6-(4-Allyloxy-styryl)-1-methansulfonyloxy-4-methyl-2-pyridon
42) 6-(3,5-Dichlor-4-hexyloxy-styryl)-4-methyl-1-propansulfonyloxy-2-pyridon
43) 4-Methyl-6-(2-phenyl-2-cyclohexyl-vinyl)-1-(toluol-4-sulfonyloxy)-2-pyridon
44) 1-(4-Methoxy-benzolsulfonyloxy)-4-methyl-6-(2-phenyl-hex-1-enyl)-2-pyridon
45) 3-Brom-6-(2-cyclohexyl-vinyl)-4-methyl-1-trifluormethansulfonyloxy-2-pyridon
46) 4-Ethyl-1-methansulfonyloxy-6-pent-1-enyl-2-pyridon

47) 1-Methansulfonyloxy-4-methyl-6-(2-methyl-prop-1-enyl)-2-pyridon
48) 6-Cyclooctylidenmethyl-1-methansulfonyloxy-4-methyl-2-pyridon
49) 6-Cyclopentylidenmethyl-4-methyl-1-(toluol-4-sulfonyloxy)-2-pyridon
50) 6-[2-(5-Brom-thiophen-2-yl)-vinyl]-3-methansulfonylamino-1-methansulfonyloxy-4-methyl-2-pyridon
51) 6-[2-(5-Brom-thiophen-2-yl)-vinyl]-3-isobutyrylamino-4-methyl-1-(toluol-4-sulfonyloxy)-2-pyridon
52) 1-Ethansulfonyloxy-6-(2-methyl-6-phenyl-hexa-1,3,5-trienyl)-4-methyl-2-pyridon
53) 6-(4-Cyclohexyloxy-styryl)-1-(1,1,2,3,3,3-hexafluor-propansulfonyloxy)-4-methyl-2-pyridon
54) 6-(3-Cyclopropyloxy-styryl)-1-methansulfonyloxy-4-methyl-2-pyridon
55) 6-(2-Benzofuran-2-yl-vinyl)-4-methyl-1-(toluol-4-sulfonyloxy)-2-pyridon
56) 6-[2-(5-Brom-thiophen-2-yl)-vinyl]-4-methyl-1-(3-trifluormethyl-benzolsulfonyloxy)-2-pyridon
57) 4-Methyl-6-[2-(5-methyl-furan-2-yl)-vinyl]-1-(naphthalin-2-ylsulfonyloxy)-2-pyridon
58) 4-Methyl-6-(4-nitro-styryl)-1-octansulfonyloxy-2-pyridon
59) 1-Hexadecansulfonyloxy-4-methyl-6-(4-nitrostyryl)-2-pyridon
60) 1-Methansulfonyloxy-4-methyl-6-(4-nitro-styryl)-2-pyridon
61) 6-(2,2-Di-thiophen-2-yl-viny1)-1-methansulfonyoxy-4-methyl-2-pyridon
62) 3-Cyan-6-(2,4-dimethoxy-styryl)-4-methyl-1-trifluormethansulfonyloxy-2-pyridon
63) 6-(2-Benzofuran-2-yl-vinyl)-4-methyl-3-thiocyanato-1-(toluol-4-sulfonyloxy)-2-pyridon
64) 6-(2-Benzofuran-2-yl-vinyl)-3-brom-1-methansulfonyloxy-4-methyl-2-pyridon
65) 3,5-Bis-isopropylmercaptomethyl-4-methyl-1-(4-nitro-benzolsulfonyloxy)-6-styryl-2-pyridon
66) 1-(4-Methoxy-benzolsulfonyloxy)-4-methyl-6-(2,3,4,5,6-pentafluor-styryl)-2-pyridon
67) 6-(3,5-Dichlor-2,4,6-trimethoxy-styryl)-4-methyl-1-(1-trifluormethyl-2,2,2-trifluor-ethan-sulfonyloxy)-2-pyridon
68) 6-(3-Brom-2,4,6-trimethoxy-styryl)-1-methansulfonyloxy-4-methyl-2-pyridon
69) 6-(2-Ferrocenyl-vinyl)-4-methy1-1-(toluol-4-sulfonyloxy)-2-pyridon
70) 6-[2-(5-Methyl-furan-2-yl)-viny1]-4-methyl-3-nitro-1-(toluol-4-sulfonyloxy)-2-pyridon
71) 6-(4-tert.-Butyl-styryl)-4-methyl-5-nitro-1-propansulfonyloxy-2-pyridon
72) 3,5-Dinitro-6-(4-tert.-butyl-styryl)-4-methyl-1-butansulfonyloxy-2-pyridon
73) 1-Benzolsulfonyloxy-3-brom-6-(4-butoxy-styryl)-4-methyl-5-nitro-2-pyridon
74) 1-(4-Brom-benzolsulfonyloxy)-4-methyl-6-(4-trifluormethyl-styryl)-2-pyridon
75) 6-(3-Chlor-4-phenyl-buta-1,3-dieny1)-1-isopropansulfonyloxy-4-methyl-2-pyridon
76) 1-Benzolsulfonyloxy-4-methyl-6-[2-(1-benzolsulfonyl-pyrrol-2-yl)-vinyl]-2-pyridon
77) 6-(10,11-Dihydro-dibenzo[a,d]cyclohept-5-ylidenmethyl)-4-methyl-1-(2-thiophen-2-ylsulfonyloxy)-2-pyridon
78) 1-Benzolsulfonyloxy-4-methy1-6-(4-styryl-styryl)-2-pyridon
79) 1-Methansulfonyloxy-4-methy1-6-(2-[1]naphthylvinyl)-2-pyridon
80) 1-Methansulfonyloxy-4-methyl-6-(4-phenyl-buta-1,3-dienyl)-2-pyridon
81) 6-(4-Dimethylamino-styry1)-1-methansu1fonyloxy-4-methyl-2-pyridon
82) 1-Methansulfonyloxy-6-(4-methoxy-styryl)-4-methyl-2-pyridon
83) 1-(4-Chlor-benzolsulfonyloxy)-6-(4-methoxy-styryl)-4-methyl-2-pyridon
84) 1-(4-Chlor-benzolsulfonyloxy)-6-(4-chlor-styryl)-4-methyl-2-pyridon
85) 1-Ethansulfonyloxy-6-(4-methoxy-styryl)-4-methyl-2-pyridon
86) 3-Brom-1-methansulfonyloxy-6-(4-methoxy-styryl)-4-methyl-2-pyridon
87) 1-Isopropansulfonyloxy-6-(4-methoxy-styryl)-4-methyl-2-pyridon
88) 1-Butansulfonyloxy-6-(4-methoxy-styryl)-4-methyl-2-pyridon
89) 6-(4-Methoxy-styryl)-4-methyl-1-trifluormethansulfonyloxy-2-pyridon
90) 6-(4-Methoxy-styryl)-1-(1,1,2,3,3,3-hexafluor-propansulfonyloxy)-4-methyl-2-pyridon
91) 6-(4-Methoxy-styryl)-4-methyl-1-benzolsulfonyloxy-2-pyridon
92) 1-(4-Fluor-benzolsulfonyloxy)-6-(4-methoxy-styryl)-4-methyl-2-pyridon
93) 6-(4-Methoxy-styryl)-1-(4-trifluor-benzolsulfonyloxy)-4-methyl-2-pyridon
94) 1-Benzolsulfonyloxy-6-(4-methoxy-styryl)-4-methyl-2-pyridon
95) 6-(4-Chlor-styryl)-1-ethansulfonyloxy-4-methyl-2-pyridon
96) 3-Brom-6-(4-chlor-styryl)-1-methansulfonyloxy-4-methyl-2-pyridon
97) 6-(4-Chlor-styryl)-1-isopropansulfonyloxy-4-methyl-2-pyridon
98) 1-Butansulfonyloxy-6-(4-chlor-styryl)-4-methyl-2-pyridon
99) 6-(4-Chlor-styryl)-4-methyl-1-trifluormethansulfonyloxy-2-pyridon
100) 6-(4-Chlor-styryl)-1-(1,1,2,3,3,3-hexafluorpropansulfonyloxy)-4-methyl-2-pyridon
101) 1-Benzolsulfonyloxy-6-(4-chlor-styryl)-4-methyl-2-pyridon
102) 6-(4-Chlor-styryl)-1-(4-fluor-benzolsulfonyloxy)-4-methyl-2-pyridon

103) 6-(4-Chlor-styryl)-4-methyl-1-(4-trifluormethylbenzol-sulfonyloxy)-2-pyridon
104) 1-Benzolsulfonyloxy-6-(4-chlor-styryl)-4-methyl-2-pyridon
105) 1,4-Bis-(4-methyl-2-oxo-6-styryl-2H-pyridin-1-yloxysulfonyl)-butan
106) 1,3-Bis-[6-(4-methoxy-styryl)-4-methyl-2-oxo-2H-pyridin-1-yloxysulfonyl]-benzol
107) 1,3-Bis-[3-brom-6-(4-chlor-styryl)-4-methyl-2-oxo-2H-pyridin-1-yloxysulfonyl]-2,4,6-trimethyl-benzol
108) 1,5-Bis-[6-(4-fluor-styryl)-4-methyl-2-oxo-2H-pyridin-1-yloxysulfonyl]-naphthalin
109) 2-Chlor-3,5-bis-[4-methyl-6-(4-methyl-styryl)-2-oxo-2H-pyridin-1-yloxysulfonyl]-thiophen

Anwendungsbeispiel 1

Dieses Beispiel demonstriert die Eignung der erfindungsgemäßen Verbindungen als Säurebildner in einer positiv arbeitenden Kopierschicht für Flachdruckplatten.

Eine Aluminiumplatte mit einer mechanisch aufgerauhten und vorbehandelten Oberfläche wurde mit einer Lösung von

| | |
|---|---|
| 6,50 Gt | eines Kresol-Formaldehyd-Novolaks (Schmelzbereich 105 bis 120°C nach DIN 53181), |
| 2,20 Gt | eines Acetals aus Piperonal und Phenoxyethanol, |
| 0,33 Gt | 6-(4-Chlor-styryl)-1-methansulfonyloxy-4-methyl-2-pyridon und |
| 0,05 Gt | Kristallviolett-Base in |
| 30,0 Gt | Ethylenglykol-monomethylether, |
| 52,0 Gt | Tetrahydrofuran und |
| 10,0 Gt | Butylacetat |

so beschichtet, daß die Schichtdicke nach dem Trocknen etwa 2 µm betrug. Die beschichtete Platte wurde durch eine Vorlage, die neben Strich- und Rastermotiven einen Halbtonstufenkeil mit 13 Stufen der optischen Dichte von je 0,15 enthielt, unter einer 5-kW-Metallhalogenid-Lampe im Abstand von 110 cm 20 Sekunden belichtet und nach einer Wartezeit von 15 Minuten mit einem Entwickler nachstehender Zusammensetzung 1 Minute entwickelt:

| | |
|---|---|
| 5,5 Gt | Natriummetasilikat x 9 $H_2O$, |
| 3,4 Gt | Trinatriumphosphat x 12 $H_2O$, |
| 0,4 Gt | Natriumdihydrogenphosphat (wasserfrei) und |
| 90,7 Gt | entionisiertes Wasser. |

Es wurde ein originalgetreues positives Abbild der Vorlage erhalten, in dem auch kleinste Details exakt wiedergegeben waren. Die so erhaltene Druckform wurde in eine Bogenoffsetmaschine eingespannt und ergab mehr als 65.000 Drucke ausgezeichneter Qualität.

Anwendungsbeispiel 2

Dieses Beispiel demonstriert die Verwendbarkeit der erfindungsgemäßen Verbindungen als Säurebildner für negativ arbeitende Photolacke in der Mikroelektronik.

Es wurde eine Beschichtungslösung hergestellt aus

| | |
|---|---|
| 8,0 Gt | des in Anwendungsbeispiel 1 genannten Kresol-Formaldehyd-Novolaks |
| 2,0 Gt | Hexamethoxymethylmelamin und |
| 0,4 Gt | 6-(4-Methoxy-styryl)-4-methyl-1-(4-nitrobenzolsulfonyloxy)-2-pyridon in |
| 56 Gt | propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.500 Umdrehungen aufgeschleudert. Nach 1 Minute Trocknen bei 100°C auf der hot plate wurde eine Schichtdicke von 1,05 µm erhalten.

Das Aufzeichnungsmaterial wurde bildmäßig unter einer Vorlage, die Strukturen bis herab zu 0,2 µm enthielt, mit der Strahlung einer Xenon-Quecksilberdampflampe (Filter mit Transmission von 360 bis 370 nm) mit einer Energie von 80 $mJ/cm^2$ belichtet. Der belichtete Wafer wurde anschließend 1 Minute bei

100°C nacherwärmt.

Die Entwicklung erfolgte mit einem wäßrigen Entwickler, der 2,38% Tetramethylammoniumhydroxid enthielt.

Nach einer Entwicklungsdauer von 120 s erhielt man ein fehlerfreies Abbild der Maske mit steilen Resistflanken, wobei Strukturen bis herab zu 0,45 µm detailgetreu aufgelöst waren. Eine Untersuchung der Flanken der Resistprofile mit Rasterelektronenmikroskopie belegte, daß diese praktisch senkrecht zur Substratoberfläche ausgerichtet waren und keinen Unterschnitt zeigten.

Anwendungsbeispiel 3

Dieses Beispiel demonstriert die Verwendbarkeit der erfindungsgemäßen Verbindungen als UV2-empfindliche Säurebildner für positiv arbeitende Photolacke in der Mikroelektronik.

Es wurde eine Beschichtungslösung hergestellt aus

| | |
|---|---|
| 8,0 Gt | eines Homopolymeren aus 3-Methyl-4-hydroxystyrol mit einem mittleren Molekulargewicht von 22.000, |
| 2,0 Gt | des in Anwendungsbeispiel 1 beschriebenen Acetals und |
| 0,4 Gt | 4-Methyl-6-styryl-1-trichlormethansulfonyloxy-2-pyridon in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.200 Umdrehungen aufgeschleudert. Nach 1 Minute Trocknen bei 100°C auf der hot plate wurde eine Schichtdicke von 1,02 µm erhalten.

Das Aufzeichnungsmaterial wurde bildmäßig unter einer Vorlage mit der Strahlung einer Xenon-Quecksilberdampflampe (Filter mit Transmission von 240 bis 260 nm) mit einer Energie von 80 $mJ/cm^2$ belichtet und vor der Entwicklung 1 Minute auf der hot plate auf 95°C erwärmt.

Entwickelt wurde das Aufzeichnungsmaterial mit einem wäßrig alkalischen Entwickler, der 2,38 Gew.-% Tetramethylammoniumhydroxid enthielt.

Nach einer Entwicklungsdauer von 90 s erhielt man ein fehlerfreies Abbild der Maske, wobei auch Strukturen $< 0,4$ µm detailgetreu aufgelöst waren. Eine Untersuchung der Flanken der Resistprofile mit Rasterelektronenmikroskopie ergab auch hier, daß diese praktisch senkrecht zur Substratoberfläche ausgerichtet waren und bis zu dieser Auflösung kein Deckschichteffekt beobachtet wurde.

Anwendungsbeispiel 4

Dieses Beispiel demonstriert die Verwendbarkeit der erfindungsgemäßen Verbindungen als lichtempfindliche Photoinitiatoren für radikalisch polymerisierbare Photolacke für gedruckte Schaltungen.

Eine Beschichtungslösung aus

| | |
|---|---|
| 25,0 Gt | eines Mischpolymerisats aus 30 Gt Methacrylsäure, 60 Gt n-Hexyl-methacrylat und 10 Gt Styrol, |
| 16,5 Gt | eines Umsetzungsprodukts aus 1 mol 2,2,4-Trimethylhexamethylen-diisocyanat und 2 mol Hydroxymethyl-methacrylat, |
| 1,5 Gt | Triethylenglykoldimethacrylat, |
| 0,7 Gt | 1-(4-Brom-benzolsulfonyloxy)-4-methyl-6-(4-trifluormethyl-styryl)-2-pyridon und |
| 0,1 Gt | 2,4-Dinitro-6-chlor-2'-acetylamino-5'-methoxy-4'-(N-β-hydroxyethyl-N-β'-cyanethyl-amino)-azobenzol in |
| 60 Gt | Methylethylketon |

wurde auf eine Polyethylenterphthalatfolie zu einem Trockenschichtgewicht von 25 $g/m^2$ aufgetragen. Dieses Zweischichtensystem wurde in einem handelsüblichen Laminator bei 120°C auf einen Träger aus Isolierstoffmaterial, der eine 35 µm dicke Kupferschicht trug, laminiert. Das Material wurde 60 Sekunden durch eine Vorlage belichtet, die neben Strich- und Rastermotiven einen Halbtonstufenkeil enthielt, wobei die in Anwendungsbeispiel 1 beschriebene Lichtquelle eingesetzt wurde. Das belichtete Material wurde mit

einer 0,8 %igen Natriumcarbonatlösung entwickelt. Es wurde ein Negativ des Strich- und Rastermotivs erhalten. Die Stufen 1 bis 6 des Halbtonstufenkeils blieben als erhabenes Relief stehen, die Stufe 7 war sichtbar angegriffen. Das freigelegte Kupfer wurde mit einer Eisen-III-chlorid-Lösung geätzt, wobei die Resistschicht nicht merklich angegriffen wurde.

## Patentansprüche

**1.** 1-Sulfonyloxy-2-pyridone der allgemeinen Formel I,

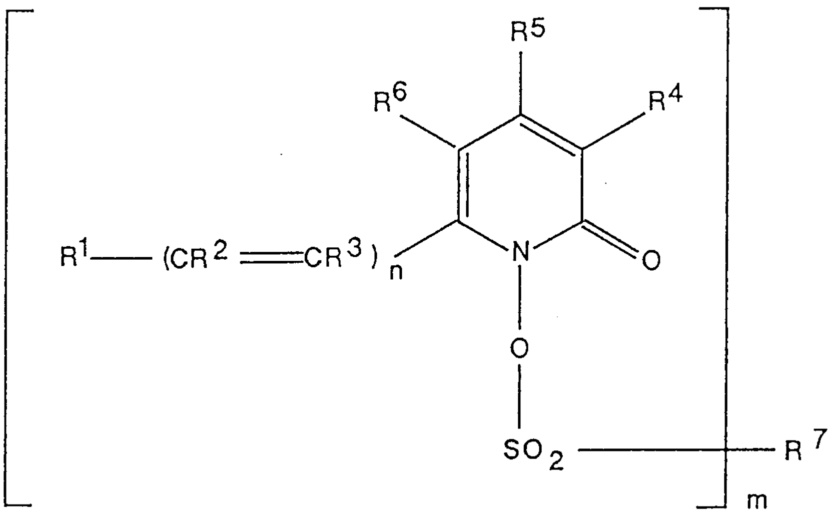

(I)

worin

| | |
|---|---|
| $R^1$ | einen Alkyl-, Cycloalkyl-, Aryl-, Aralkenyl-, Heteroaryl- oder Heteroaralkenylrest, |
| $R^2$ | Wasserstoff, Chlor, Brom, einen Alkyl-, Cycloalkyl-, Aryl- oder Heteroarylrest oder |
| $R^1$ und $R^2$ | gemeinsam einen fünf- bis achtgliedrigen Ring ausbilden, |
| $R^3$ | Wasserstoff oder einen Alkylrest, |
| $R^4$ | Wasserstoff, Halogen, Nitro, Acylamino, Cyan, Thiocyanato, einen Alkyl-, Alkenyl-, Alkinyl-, Aryl-, Aralkyl-, Alkylthio-, Arylthio- oder Cycloalkylthiorest, |
| $R^5$ | Wasserstoff, einen Alkyl- oder Arylrest oder |
| $R^4$ und $R^5$ | zusammen einen fünf- bis achtgliedrigen Ring ausbilden, |
| $R^6$ | Wasserstoff, Halogen, Nitro, Acylamino, Cyan, Thiocyanato, einen Alkyl-, Alkenyl-, Alkoxyalkyl-, Aryl-, Aralkyl-, Alkylthio-, Cycloalkylthio- oder Arylthiorest, |
| $R^7$ | einen Alkyl-, Cycloalkyl, per- oder hochfluorierten Alkylrest oder einen Aryl-, Arylalkyl- oder Heteroarylrest oder einen Alkylen- oder Arylenrest |
| m | die Zahl 1 oder 2 und |
| n | eine ganze Zahl von 1 bis 3 |

bedeuten.

**2.** 1-Sulfonyloxy-2-pyridone gemäß Anspruch 1, dadurch gekennzeichnet, daß $R^1$ ein Alkyl- oder Cycloalkylrest oder ein Arylrest der allgemeinen Formel II

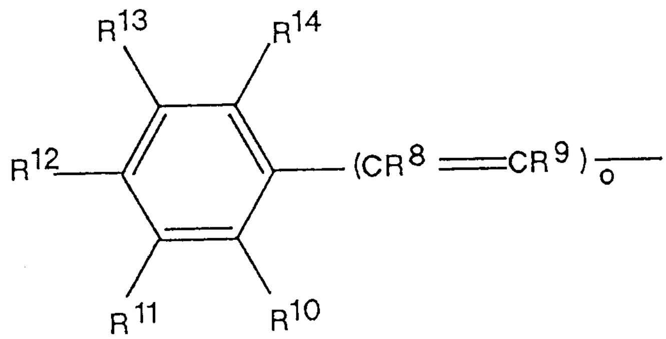

(II)

ist, worin

| | |
|---|---|
| $R^8$ und $R^9$ | gleich oder verschieden sind und Wasserstoff, eine niedere Alkyl- oder Arylgruppe darstellen, |
| $R^{10}$ bis $R^{14}$ | gleich oder verschieden sind und Wasserstoff, einen Alkyl-, Alkenyl-, Alkoxy-, Alkylthio-, Alkansulfonylrest mit jeweils bis zu 6 Kohlenstoffatomen, einen Cycloalkyloxy-, Cycloalkylthio-, Cycloalkansulfonylrest mit bis zu 8 Kohlenstoffatomen, gegebenenfalls am aromatischen Ring substituierten Phenyl-, Styryl-, Phenoxy-, Phenylthio-, Benzolsulfonyl-, Phenylalkoxy-, Phenylalkylthio-, Phenylalkansulfonylrest mit bis zu 3 Kohlenstoffatomen in der Alkylkette, Hydroxy, Halogen, Trifluoromethyl, Nitro, Cyan, Alkoxycarbonyl, Carbamoyl, das gegebenenfalls am Stickstoff durch einen oder zwei Alkylrest(e), die gegebenenfalls zu einem 5- bis 7-gliedrigen Ring verbunden sind, substituiert ist, Sulfamoyl, das gegebenenfalls am Stickstoff durch ein oder zwei Alkylrest(e), die gegebenenfalls zu einem 5- bis 7-gliedrigen Ring verbunden sind, substituiert ist, Alkansulfonyloxy, Arylsulfonyloxy, Acylamino, Alkylamino oder Arylamino bedeuten, oder zwei der Substituenten $R^{10}$ bis $R^{14}$, die einander benachbart sind, einen oder zwei weitere ankondensierte Ring(e) bilden, und |
| o | für die Zahl 0 oder 1 steht. |

**3.** 1-Sulfonyloxy-2-pyridone gemäß den Ansprüchen 1 oder 2, dadurch gekennzeichnet, daß $R^1$ ein 5- oder 6-gliedriger Heterocyclus mit bis zu drei Heteroatomen der allgemeinen Formel III

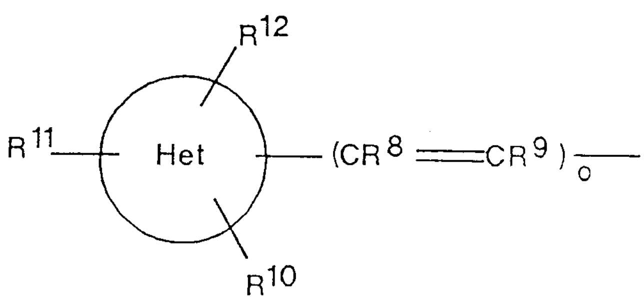

(III)

ist.

**4.** 1-Sulfonyloxy-2-pyridone gemäß den Ansprüchen 1 bis 3, dadurch gekennzeichnet, daß in der allgemeinen Formel I

| | |
|---|---|
| $R^1$ | ein Ferrocenylrest und |
| $R^2$ | Wasserstoff, Chlor, Brom, Alkyl-, Cycloalkyl oder ein Rest der allgemeinen Formeln II oder III ist oder |
| $R^1$ und $R^2$ | gemeinsam einen fünf- bis achtgliedrigen Ring ausbilden, |
| $R^3$ | Wasserstoff oder ein Alkylrest, |
| $R^4$ | Wasserstoff, Halogen, Nitro, Acylamino, Cyan, Thiocyanato, ein Alkyl-, Aryl-, Alkylthio-, Arylthio- oder Cycloalkylthiorest, |

| | |
|---|---|
| $R^5$ | Wasserstoff, ein Alkyl- oder Arylrest ist oder |
| $R^4$ und $R^5$ | gemeinsam einen fünf- bis achtgliedrigen Ring ausbilden, |
| $R^6$ | Wasserstoff, Halogen, Nitro, Acylamino, Cyan, Thiocyanato, ein Alkyl-, Aryl-, Alkylthio-, Arylthio- oder Cycloalkylthiorest, |
| $R^7$ | ein Alkyl-, Cycloalkyl-, per- oder hochfluorierten Alkylrest oder ein Aryl-, Arylalkyl-, Heteroarylrest oder ein Alkylen- oder Arylenrest ist und |
| m | für die Zahl 1 oder 2 und |
| n | für eine ganze Zahl von 1 bis 3 steht. |

**5.** 1-Sulfonyloxy-2-pyridone gemäß Anspruch 1, dadurch gekennzeichnet, daß $R^7$ ein Alkylrest mit 1 bis 4 Kohlenstoffatomen, ein hoch- oder perfluorierter Alkylrest mit 1 bis 4 Kohlenstoffatomen oder ein Arylrest der allgemeinen Formel IV

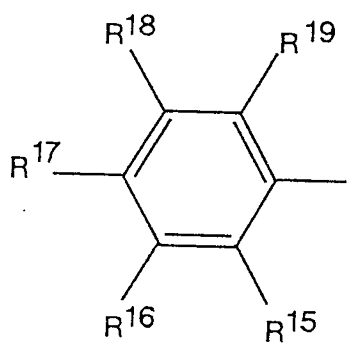

(IV)

ist, worin $R^{15}$ bis $R^{19}$ gleich oder verschieden sind und Wasserstoff- oder Halogenatome, die vorzugsweise Fluor, Chlor oder Brom sind, Alkylreste mit bis zu 6 Kohlenstoffatomen, die unsubstituiert oder durch Halogenatome, vorzugsweise Chlor oder Brom, Aryl- oder Aryloxyreste, substituiert sind und in denen einzelne Methylengruppen durch Sauerstoff- oder Schwefelatome ersetzt sein können und wobei jeweils zwei dieser Reste unter Ausbildung eines 5- oder 6-gliedrigen Rings verknüpft sein können, Cycloalkylreste mit bis zu 8 Kohlenstoffatomen, Alkenylreste mit bis zu 6 Kohlenstoffatomen, Aryl- oder Aryloxyreste mit bis zu 10 Kohlenstoffatomen bedeuten und die Gesamtzahl der Kohlenstoffatome der Reste $R^{15}$ bis $R^{19}$ maximal 12 beträgt.

**6.** 1-Sulfonyloxy-2-pyridone gemäß Anspruch 1, dadurch gekennzeichnet, daß $R^7$ ein Naphthyl- oder Heteroarylrest mit bis zu 10 Kohlenstoffatomen, ein Alkylenrest mit bis zu 6 Kohlenstoffatomen oder ein Arylen- oder Heteroarylenrest mit bis zu 14 Kohlenstoffatomen ist.

**7.** 1-Sulfonyloxy-2-pyridone gemäß Anspruch 1, dadurch gekennzeichnet, daß

| | |
|---|---|
| $R^2$, $R^3$, $R^4$ und $R^6$ | ein Wasserstoffatom, |
| $R^5$ | eine Methylgruppe, |
| $R^7$ | einen Methyl-, Ethyl-, Trifluormethyl-, 1,1,2,3,3,3-Hexafluorpropyl-, Phenyl-, Tolyl-, 4-Fluorphenyl-, 4-Chlorphenyl-, 4-Bromphenyl oder 4-Nitrophenylrest und |
| m und n | jeweils die Zahl 1 |

bedeuten.

**8.** Verfahren zur Herstellung von 1-Sulfonyloxy-2-pyridonen gemäß einem oder mehreren der Ansprüche 1 bis 7, dadurch gekennzeichnet, daß man

a) ein 6-Halogenomethyl-2-pyron in eine Phosphonium- oder Phosphonoverbindung überführt,

b) diese einer Wittig-Reaktion oder deren Varianten unterwirft,

c) die erhaltene Verbindung in ein 1-Hydroxy-2-pyridon umwandelt und

d) mit einem Sulfonsäurehalogenid zu einem 1-Sulfonyloxy-2-pyridon der allgemeinen Formel I umsetzt.

**9.** Verfahren gemäß Anspruch 8, dadurch gekennzeichnet, daß das Sulfonsäurehalogenid ein Sulfonsäurechlorid ist.

**10.** Verfahren gemäß den Ansprüchen 8 oder 9, dadurch gekennzeichnet, daß die Umsetzung mit dem Sulfonsäurehalogenid in Gegenwart einer organischen Base durchgeführt wird.

**11.** Verfahren gemäß einem oder mehreren der Ansprüche 8 bis 10, dadurch gekennzeichnet, daß die Umsetzung mit dem Sulfonsäurehalogenid in einem organischen Lösemittel bei Temperaturen zwischen 0 und 20°C durchgeführt wird.

**12.** Verwendung der 1-Sulfonyloxy-2-pyridone gemäß einem oder mehreren der Ansprüche 1 bis 7 zur Herstellung strahlungsempfindlicher Gemische und Aufzeichnungsmaterialien.

Europäisches Patentamt

# EUROPÄISCHER RECHERCHENBERICHT

Nummer der Anmeldung

EP 92 10 6138

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 330 386 (AMERICAN TELEPHONE AND TELEGRAPH COMPANY)<br>* das ganze Dokument *<br>--- | 1-12 | C07D213/89<br>G03C1/73 |
| A,D | US-A-4 425 424 (H.W. ALTLAND ET AL.)<br>* das ganze Dokument *<br>--- | 1-12 | |
| A | US-A-4 197 080 (J.D. MEE)<br>* Ansprüche 1-12; Beispiele *<br>----- | 1,12 | |

RECHERCHIERTE SACHGEBIETE (Int. Cl.5)

C07D

Der vorliegende Recherchenbericht wurde für alle Patentansprüche erstellt

| Recherchenort | Abschlußdatum der Recherche | Prüfer |
|---|---|---|
| DEN HAAG | 28 JULI 1992 | P. BOSMA |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)